# EUROPÄISCHE PATENTANMELDUNG

(11) **EP 3 461 407 A1**
(43) Veröffentlichungstag der Anmeldung: **03.04.2019**
(21) Anmeldenummer: 17193387.2
(22) Anmeldetag: 27.09.2017
(51) Int. Cl.: A61B 6/00, A61L 2/28, G16H 40/00

(54) **REINIGUNG EINES GEHÄUSES EINER MEDIZINISCHEN BILDGEBUNGSANLAGE**

(71) Anmelder: Siemens Healthcare GmbH, 91052 Erlangen (DE)
(72) Erfinder: Weingarten, Markus, 96049 Bamberg (DE)

(57) **Zusammenfassung**

Die Erfindung betrifft ein Verfahren zum Reinigen einer Gehäuseoberfläche (4) einer medizinischen Bildgebungsanlage (1) umfassend folgende Schritte:
- Aktivieren (S1) eines Reinigungsmodus (RM) an der Bildgebungsanlage,
- Detektieren (S4) eines Reinigungsvorgangs (RV) in wenigstens einem lokalen Teilbereich (10, 11, 12) der Gehäuseoberfläche, und
- Anzeigen (S5) einer Information bezüglich des wenigstens einen, lokalen Teilbereichs, der gereinigt wurde.

Die Erfindung betrifft auch eine medizinische Bildgebungsanlage, eine Recheneinheit, ein Computerprogramm sowie einen computerlesbaren Datenträger.

## Beschreibung

Die Erfindung betrifft ein Verfahren zum Reinigen einer Gehäuseoberfläche einer medizinischen Bildgebungsanlage, eine entsprechende medizinische Bildgebungsanlage, eine Recheneinheit, entsprechendes Computerprogramm sowie entsprechenden computerlesbaren Datenträger. Bei der medizinischen Bildgebungsanlage handelt es sich insbesondere um ein C-Bogen-Röntgen-Gerät, wie es bspw. in der Angiographie zum Einsatz kommt.

Medizinische Einrichtungen wie z.B. Krankenhäuser, Ärzte-Zentren oder Radiologie-Zentren unterliegen strengen Hygiene-Vorschriften, um die Ausbreitung von Krankheitserregern und Keimen zu vermindern bzw. zu verhindern. Die Hygiene-Vorschriften, entsprechen einem Standard. Sie regeln unter anderem, wie Räume, Einrichtungsgegenstände, medizinische Instrumente, Geräte oder Anlagen gereinigt werden müssen, insbesondere bei häufigem und/oder direktem Patientenkontakt. Entsprechende Reinigungsvorschriften gelten auch für medizinische Bildgebungsanlagen.

Bei Hersteller-seitiger Auslieferung einer medizinischen Bildgebungsanlage wird zudem ein Reinigungsplan ausgehändigt. Dieser umfasst eine detaillierte Liste und Anleitung, welche Komponenten der Anlage wie zu reinigen sind.

Heutzutage unterliegen medizinische Einrichtungen immer mehr dem Wettbewerb und müssen sich anhand ihrer Wirtschaftlichkeit messen lassen. Dies führt vermehrt zu einer Auslagerung von Reinigungsdienstleistungen an externe Drittanbieter, deren Mitarbeiter ggf. nur ungenügend qualifiziert sind und unter hohem Zeit- und Kostendruck arbeiten. So muss bspw. die Reinigung eines gesamten C-Bogen-Röntgensystems binnen drei Minuten abgeschlossen sein. Insbesondere bei intra-operativ eingesetzten Anlagen mit ggf. stärkerer Verschmutzung ergibt sich unter genannten Rahmenbedingungen ein erhöhtes Gefährdungspotential, wenn die Reinigung nur unvollständig und/oder ungründlich erfolgen kann.

Demgegenüber ist es Aufgabe der vorliegenden Erfindung, alternative Mittel bereit zu stellen, die es erlauben, eine medizinische Bildgebungsanlage entsprechend den Hygiene-Vorschriften zuverlässig, reproduzierbar und gründlich zu reinigen. Insbesondere ist es Aufgabe der vorliegenden Erfindung, Mittel bereits zu stellen, die selbst unter Zeitdruck und/oder mangelnder Qualifikation des Reinigungspersonals ein hochwertiges Reinigungsergebnis sicherstellen.

Diese Aufgabe wird gelöst durch ein Verfahren zum Reinigen einer Gehäuseoberfläche einer medizinischen Bildgebungsanlage, eine medizinische Bildgebungsanlage, eine Recheneinheit, ein Computerprogramm sowie einen computerlesbaren Datenträger gemäß den unabhängigen Ansprüchen. Bevorzugte und/oder alternative, vorteilhafte Ausgestaltungsvarianten sind Gegenstand der abhängigen Ansprüche.

Nachstehend wird die erfindungsgemäße Lösung der Aufgabe in Bezug auf das beanspruchte Verfahren als auch in Bezug auf die beanspruchten Vorrichtungen beschrieben. Hierbei erwähnte Merkmale, Vorteile oder alternative Ausführungsformen sind ebenso auch auf die anderen beanspruchten Gegenstände zu übertragen und umgekehrt. Mit anderen Worten können gegenständliche Ansprüche (die beispielsweise auf ein Verfahren gerichtet sind) auch mit Merkmalen, die in Zusammenhang mit einer der Vorrichtungen beschrieben oder beansprucht sind, weitergebildet sein. Die entsprechenden funktionalen Merkmale des Verfahrens werden dabei durch entsprechende gegenständliche Module oder Einheiten ausgebildet.

Die vorliegende Erfindung betrifft in einem ersten Aspekt ein Verfahren zum Reinigen einer Gehäuseoberfläche einer medizinischen Bildgebungsanlage. Das Verfahren umfasst mehrere Schritte.

Der erste Schritt umfasst ein Aktivieren eines Reinigungsmodus an der Bildgebungsanlage. Entsprechend ist vorgesehen, dass die Bildgebungsanlage wenigstens einen Reinigungsmodus und einen weiteren Modus umfasst, in welchem sie betrieben werden kann. Der Reinigungsmodus ist dafür vorgesehen, eingestellt zu werden, wenn die Anlage gereinigt werden soll. Mit anderen Worten werden in einem Reinigungsmodus Komponenten der Bildgebungsanlage in eine den Reinigungsvorgang unterstützenden Zustand gebracht. Der wenigstens, eine weitere Modus könnte bspw. ein Bilddatenerfassungsmodus sein, in welchem die Bildgebungsanlage eingerichtet ist, Bilddaten, bspw. von einem Patienten, aufzunehmen. Mit anderen Worten werden in einem Bilddatenerfassungsmodus Komponenten der Bildgebungsanlage in eine die Bilddatenerfassung unterstützenden Zustand gebracht. Bspw. kann in einem Bilddatenerfassungsmodus eine Röntgenquelle zur Erzeugung und Aussendung von Röntgenstrahlen aktiviert sein. Ein Aktivieren umfasst in diesem Sinne ein Starten, Einstellen oder Einleiten des Reinigungsmodus. Insofern kann das Aktivieren auch ein Wechseln von einem Betriebsmodus in den Reinigungsmodus umfassen. Andere, weitere Modi, wie bspw. ein Wartungsmodus oder dergleichen, sind ebenfalls möglich.

Unter einer Bildgebungsanlage im Sinne der Erfindung ist bspw. eine Computertomographie-Anlage oder ein C-Bogen-Röntgen-Gerät, insbesondere umfassend einen Roboter-Stator-Arm mit verschiedenen Verstellachsen zu verstehen. Die Bildgebungsanlage kann auch als Magnetresonanz-Tomograph oder klassisches Ultraschall-Gerät ausgebildet sein. Andere Ausgestaltungsvarianten der medizinischen Bildgebungsanlage sind ebenfalls denkbar und liegen im Rahmen der hiesigen Erfindung.

Ein zweiter Verfahrensschritt umfasst ein Detektieren eines Reinigungsvorgangs in wenigstens einem lokalen Teilbereich der Gehäuseoberfläche. Der zweite Verfahrensschritt erfolgt erfindungsgemäß nach dem ersten Verfahrensschritt, also während der Reinigungsmodus der Anlage aktiviert ist bzw. nachdem dieser aktiviert wurde.

Die Gehäuseoberfläche der Bildgebungsanlage kann die gesamte äußere Oberfläche, bspw. des Gehäuses der Bildgebungsanlage oder nur einen Teil davon beschreiben. Unter Gehäuse in diesem Sinne sind nicht nur die Cover, Blenden bzw. Verkleidungen von an der Bildgebung selbst beteiligten Komponenten der Anlage zu verstehen, sondern auch derjenigen Komponenten der Anlage, die zur Interaktion mit einer Bedienperson bzw. einem Nutzer und/oder der Bewegung/einem Verstellen der Anlage oder einzelner ihrer Komponenten oder dergleichen dienen. Anders ausgedrückt kann die Gehäuseoberfläche durch alle die Anlage nach außen hin begrenzenden Oberflächen der Anlage gebildet sein. Die Gehäuseoberfläche kann als zusammenhängende, einstückige Fläche ausgebildet sein oder mehrere einzelne, nicht notwendigerweise zusammenhängende Teilflächen umfassen. Ein lokaler Teilbereich der Gehäuseoberfläche ist definiert als ein gegenüber der Gehäuseoberfläche lokal begrenzter und deutlich kleinerer Teil der Gehäuseoberfläche. Bspw. ist ein lokaler Teilbereich das Röntgenquellen-Cover oder das Cover des gegenüberliegenden Röntgendetektors einer C-Bogen-Röntgenanlage. Ein lokaler Teilbereich kann bspw. eine Fläche von 10*cm*² - 100*cm2* umfassen, im Einzelfall aber auch größer oder kleiner sein.

Gemäß dem zweiten Verfahrensschritt ist vorgesehen, dass in wenigstens einem Teilbereich der Gehäuseoberfläche ein Reinigungsvorgang detektiert, sprich, erfasst, registriert, gemessen oder erkannt wird. Dazu sind in der Gehäuseoberfläche wenigstens in dem wenigstens einen betrachteten Teilbereich entsprechende Mittel in Form von Sensorelementen vorgesehen, die für eine Detektion eines Reinigungsvorgangs ausgebildet sind, wie weiter unten näher beschrieben wird.

Unter einem erfindungsgemäßen Reinigungsvorgang ist eine mechanische und/oder chemische Behandlung mit dem Ziel der Schmutzentfernung zu verstehen. Für den Reinigungsvorgang können Hilfsmittel wie Lappen, Tücher, Wedel, Bürsten und/ oder Reinigungs- oder Desinfektionsmittel und/oder Wasser eingesetzt werden. Die Reinigung kann durch einen Nutzer, bspw. Reinigungspersonal durchgeführt werden. Bspw. kann ein Reinigungsvorgang ein Abwischen, Abstauben und/oder Abbürsten eines Teilbereichs der Gehäuseoberfläche mit einem feuchten Tuch, einem Wedel und/oder einer Bürste umfassen. Ein Reinigungsvorgang kann alternativ und/oder zusätzlich ein Verstellen einer oder mehrerer, einzelner Komponenten der Bildgebungsanlage umfassen. Bspw. kann das Reinigungspersonal bei einer Reinigung eine Komponente der Anlage verstellen, verdrehen, verkippen, verfahren oder dergleichen, insbesondere nach Abschluss eines Reinigungsvorgangs in Bezug auf einen lokalen Teilbereich der Gehäuseoberfläche oder der Komponente. Bspw. kann ein Patiententisch einer Bildgebungsanlage eine bestimmte, vorab definierte Position für den Reinigungsvorgang und eine andere bestimmte, vorab definierte Position nach Abschluss des Reinigungsvorgangs einnehmen. Ein anderes Beispiel stellt eine C-Bogen-Röntgenanlage dar, bei der der C-Bogen während der Reinigung ebenfalls zwischen verschiedenen vorab, definierten Position verstellt wird, bspw. um die Erreichbarkeit verschiedener Komponenten der Anlage für das Reinigungspersonal zu erhöhen oder bereits gereinigte Komponenten so anzuordnen, dass sie bei einer Reinigung weiterer Komponenten nicht wieder beschmutzt werden können. Das Verstellen kann hier bspw. mittels des Industrie-Roboters erfolgen. Dieser verfügt bspw. über mehrere, insbesondere drei Kipp-Gelenke, und ein Rotationsgelenk, sodass der C-Bogen über ein Verstellen der Gelenke fast jede beliebige Position einnehmen kann. Alternativ/oder zusätzlich kann der C-Bogen mittels Schlitten am Roboter-Arm angeordnet sein.

Entscheidend in Bezug auf den Reinigungsvorgang ist, dass durch diesen bzw. in Ausführung desselben eine messbare Kraft auf die Gehäuseoberfläche ausgeübt wird, wie weiter unten ausführlicher beschrieben.

Ein dritter Schritt des Verfahrens umfasst ein Anzeigen einer Information bezüglich des wenigstens einen, lokalen Teilbereichs, der gereinigt wurde. Mit anderen Worten erfolgt in diesem Schritt eine Ausgabe einer Information, die angibt, dass bzw. welcher lokale Teilbereich bereits gereinigt wurde.

Die Anzeige der Information erfolgt bevorzugt unverzüglich nach bzw. mit der Detektion des Reinigungsvorgangs. Die Anzeige der Information kann auf verschiedene Art und Weise erfolgen, bspw. akustisch mittels eines Signaltons und/oder optisch mittels einer Signalleuchte. Die Anzeige kann entsprechend über verschiedene Ausgabemedien erfolgen, bspw. Lautsprecher, eine Signallampe und/oder eine Ausgabe-Einheit der Bildgebungsanlage. Die Anzeige erfolgt bevorzugt für einen Benutzer, besonders bevorzugt das Reinigungspersonal.

Durch die erfindungsgemäße Anzeige, welcher lokale Teilbereich der Gehäuseoberfläche bereits gereinigt wurde, erfolgt vorteilhaft eine Differenzierung zwischen Teilbereichen für einen Benutzer, die schon sauber sind und Teilbereichen, die er noch reinigen muss. So wird sichergestellt, dass bei der Reinigung der Anlage keine Teilbereiche der Gehäuseoberfläche vergessen oder übersehen werden, selbst dann, wenn der Benutzer den für die Anlage vorgesehenen Reinigungsplan nicht kennt. Da die Anzeige instantan erfolgt, also sofort, nachdem ein Teilbereich gereinigt wurde, sprich, während des Reinigens der Anlage durch den Benutzer, bekommt dieser eine intuitive und somit zeitsparende Hilfestellung für die Reinigung.

Idealerweise werden der zweite und der dritte Schritt des Verfahrens in einer Wiederholschleife iterativ durchlaufen, bis eine gewünschte oder per Reinigungsplan vorgeschriebene Vielzahl von Komponenten der Bildgebungsanlage, bevorzugt jedoch die gesamte Gehäuseoberfläche der Anlage, gereinigt wurden.

In einem bevorzugten Ausführungsbeispiel der Erfindung erfolgt das Aktivieren des Reinigungsmodus durch einen Benutzer. In dieser Ausgestaltungsvariante kann der Benutzer bspw. per Knopf- bzw. Tastenbetätigung und/oder Sprach- bzw. Gestensteuerung den Reinigungsmodus für die Bildgebungsanlage starten. Der Nutzer kann so eigenständig den Zeitpunkt der anschließenden Reinigung der Anlage festlegen. Es kann vorgesehen sein, dass aus Sicherheitsgründen eine manuelle Aktivierung des Reinigungsmodus ausgeschlossen ist, wenn die Bildgebungsanlage gerade gewartet wird oder Bilddaten erfasst werden. Alternativ dazu kann, sofern möglich, der Reinigungsmodus automatisch durch die Anlage selbst gestartet werden, bspw. täglich zu einer bestimmten Uhrzeit außerhalb der üblichen Betriebszeiten der Anlage, bspw. morgens um 4:00 Uhr.

In einem Ausführungsbeispiel der vorliegenden Erfindung umfasst das Aktivieren des Reinigungsmodus ein Anzeigen eines Reinigungsplans für die Bildgebungsanlage auf einer Ausgabe-einheit der Bildgebungsanlage. Der Reinigungsplan ist eine spezifisch auf die Bildgebungsanlage ausgerichtete Anleitung, welche Komponenten der Anlage wie zu reinigen sind. Der Reinigungsplan kann vorteilhaft eine listenhafte Ausgestaltung aufweisen oder eine Schrittfolge für einzelne Reinigungsschritte umfassen. Diese Ausgestaltung dient als Hilfestellung für das Reinigungspersonal. Über die Anzeige des Reinigungsplans hat der Benutzer die Möglichkeit, sich den Reinigungsprozess in seiner Gesamtheit zu vergegenwärtigen oder zu rekapitulieren. Der Reinigungsplan kann unterstützend zusammen mit einer Piktogramm-artigen Übersichtsdarstellung der Bildgebungsanlage über die Ausgabeeinheit angezeigt werden, in welcher einzelne Reinigungsschritte veranschaulicht werden können. Besonders bevorzugt ist die Anzeige des Reinigungsplans interaktiv ausgebildet. Der Reinigungsplan wird dann über die Ausgabeeinheit während des gesamten Reinigungsprozesses für das Reinigungspersonal dargestellt und darin entsprechend dem dritten Schritt des erfindungsgemäßen Verfahrens angezeigt, welcher lokale Teilbereich schon gereinigt wurde, bzw. welcher Reinigungsschritt schon erledigt wurde.

Wie oben schon angedeutet, umfasst der Reinigungsvorgang eines lokalen Teilbereichs der Gehäuseoberfläche, dass eine Kraft auf die Gehäuseoberfläche ausgeübt wird, sei es durch eine Putzbewegung entlang bzw. auf der Gehäuseoberfläche oder durch ein Verstellen der Anlage oder einzelner ihrer Komponenten beim Reinigen. Entsprechend umfasst das Detektieren des Reinigungsvorgangs in einem weiteren Ausführungsbeispiel, mittels wenigstens eines in räumlicher Nähe zu der Gehäuseoberfläche des lokalen Teilbereichs angeordneten Sensorelements, diese auf die Gehäuseoberfläche ausgeübte Kraft zu detektieren, zu messen, zu erfassen oder dergleichen. Bei einem Sensorelement im Sinne der vorliegenden Erfindung kann es sich grundsätzlich um einen beliebigen Sensor handeln, der eingerichtet ist, eine auf ihn wirkende Kraft zu detektieren. Dabei kann die Krafteinwirkung in nur eine oder verschiedene Raumrichtungen berücksichtigt werden. Genauer gesagt ist das Sensorelement eingerichtet, eine der einwirkenden Kraft entsprechende Kenngröße zu detektieren. Fern muss das Sensorelement ausgebildet sein, in, an, auf, und/oder unter der Gehäuseoberfläche der Bildgebungsanlage verbaut, angeordnet bzw. befestigt zu werden. In einem lokalen Teilbereich der Gehäuseoberfläche ist jeweils wenigstens ein Sensorelement vorgesehen. Vorteilhaft umfasst ein lokaler Teilbereich, bspw. in Abhängigkeit seiner Größe, eine Vielzahl von Sensorelementen. Je größer die Flächendichte der Sensorelemente ist, umso größer ist die erfindungsgemäße Sicherstellung einer gründlichen Reinigung der Anlage. Die Flächendichte der Sensorelemente kann über die gesamte Gehäusefläche der Bildgebungsanlage variieren. Vorteilhaft ist die Flächendichte der Sensorelemente in den lokalen Teilbereichen der Gehäuseoberfläche vergleichbar hoch, bei denen eine gründliche Reinigung besonders kritisch ist. Vorteilhaft ist also die gesamte Gehäuseoberfläche einer Bildgebungsanlage mit Sensorelementen ausgestattet. Ferner kann erfindungsgemäß vorgesehen sein, dass eine Bildgebungsanlage verschiedenartige Sensorelemente umfasst, die jeweils zum Detektieren verschiedener die einwirkende Kraft repräsentierende Kenngrößen ausgebildet sind.

Gemäß einem weiteren bevorzugten Ausführungsbeispiel umfasst das Detektieren, eine der durch den Reinigungsvorgang ausgeübten Kraft entsprechende Kenngröße aus der Gruppe der folgenden Kenngrößen zu erfassen: Druck, Spannung, Strom, Kapazität, Widerstand, Kraft, Drehmoment. Weitere, nicht genannte Kenngrößen sind ebenfalls möglich und im Sinne der Erfindung. Das Sensorelement kann folglich bspw. als kapazitiver Sensor, als Metall- oder Halbleiterdehnungsmessstreifen, als Force Sensing Resitor, als piezoelektrischer, magnetoelastischer Sensor oder als Kombination verschiedene physikalische Effekte ausnutzender Sensoren ausgebildet sein. Die Ausgestaltung eines individuellen Sensorelements ist insbesondere abhängig von der Lage des lokalen Teilbereichs innerhalb der Gehäuseoberfläche und/oder der dort zu erwartenden Krafteinwirkung durch den Reinigungsprozess.

Bspw. kann die Gehäuseoberfläche, also das Cover einer Anlage Dehnungsmessstreifen umfassen. Dazu kann das Cover als geschäumtes Bauteil ausgebildet sein, in welches an den entsprechenden Stellen die Messstreifen eingeschäumt wurden. In einem weiteren Beispiel können die Gelenke eines Stator-Roboters einer C-Bogen-Röntgen-Anlage mit Drehmomenten-Sensoren ausgestattet sein.

Insbesondere können die Sensorelemente derart ausgebildet sein, dass sie in einem anderen Betriebsmodus zur Einhaltung von Sicherheitsvorschriften dienen können. Bspw. kann in einem Bilddatenerfassungsmodus vorgesehen sein, dass die Sensorelemente bei Detektion einer einwirkenden Kraft, bspw. durch willkürliches Zusammentreffen einer Person oder von weiterer, im Untersuchungsraum befindlicher Ausrüstung, unverzüglich eine parallel ausgeführte Bewegung der Bildgebungsanlage bzw. einer ihrer Komponenten stoppen, um Verletzungen und/oder Beschädigungen zu vermeiden.

Wie eingangs schon erwähnt, erfolgt das Anzeigen der Information bezüglich des gereinigten lokalen Teilbereichs in einem Ausführungsbeispiel der Erfindung optisch. Dies ermöglicht eine bevorzugt intuitive und schnelle Veranschaulichung des Reinigungsfortschrittes, insbesondere für ungeschultes Reinigungspersonal.

In einer weiteren Ausführungsform der Erfindung erfolgt das optische Anzeigen der Information bezüglich des gereinigten lokalen Teilbereichs über die Gehäuseoberfläche und/oder über die Ausgabeeinheit der Bildgebungsanlage. Das Anzeigen kann also über die Ausgabeeinheit der Bildgebungsanlage, insbesondere im Rahmen der Anzeige eines Reinigungsplans erfolgen. Bspw. kann dafür ein Abschluss eines Reinigungsschritts bzw. der Abschluss der Reinigung eines lokalen Teilbereichs durch farbliche Hervorhebung des entsprechenden Listenpunktes, farbliche Markierung des lokalen Teilbereichs in dem parallel angezeigten Piktogramm der Bildgebungsanlage oder durch ein Icon, bspw. in Form eines Häkchens an geeigneter Stelle, angezeigt werden. Alternative Darstellungen des Reinigungsabschlusses über die Ausgabeeinheit sind ebenfalls denkbar. In einer alternativen oder zusätzlichen Ausgestaltung der Erfindung erfolgt die optische Anzeige über die Gehäuseoberfläche selbst. Dazu können in der Gehäuseoberfläche zusätzlich zu den Sensorelementen Leuchtelemente vorgesehen sein, die bei detektiertem Reinigungsvorgang für einen lokalen Teilbereich aktiviert werden oder ihre Farbe wechseln. Ein Leuchtelement kann so bspw. als OLED, LED oder vergleichbares Leuchtmittel ausgebildet sein. Für jeden lokalen Teilbereich ist wenigstens ein Leuchtelement, bevorzugt jedoch mehrere Leuchtelemente vorgesehen, bspw. kann die Anzahl der Leuchtelemente zu der Anzahl der Sensorelemente in einem lokalen Teilbereich korrespondieren. Die Leuchtelemente können in direkter Nachbarschaft, bspw. in einem Abstand von wenigen Zentimetern, insbesondere 2cm bis 8cm angeordnet sein, sodass sich aus der Position des Leuchtelements der Anzeige unzweifelhaft ergibt, dass diese bereits geputzt wurde. Alternativ kann eine bevorzugte Position bzw. ein bevorzugter Bereich in der Gehäuseoberfläche eine Ansammlung von Leuchtelementen für verschiedene lokale Teilbereiche umfassen. Diese Position bzw. dieser Bereich ist für das Reinigungspersonal vorteilhaft besonders leicht einsehbar.

In einer weiteren bevorzugten Ausführungsform der Erfindung ist vorgesehen, dass der Betrieb der Bildgebungsanlage im Reinigungsmodus eine Bedienbarkeit der Bildgebungsanlage im Sinne einer Bilddatenerfassung sperrt. Mit anderen Worten können mit einer Bildgebungsanlage keine Bilddaten erfasst werden, wenn ihr Reinigungsmodus aktiviert wurde. Anders ausgedrückt, schließt ein Reinigungsmodus Funktionalitäten der Bildgebungsanlage bzw. ihrer Komponenten oder Einzelbestandteile, die bei der Bilddatenerfassung eingeschaltet bzw. aktiv sind, aus. Dadurch wird sichergestellt, dass das Reinigungspersonal keiner von der Bildgebung ausgehenden Gefahr, bspw. Röntgenstrahlung, ausgesetzt ist. Insbesondere bedeutet dies auch, dass bspw. die Sensorelemente, die zur Einhaltung von Sicherheitsvorgaben im Bilddatenerfassungsmodus zur Feststellung von Krafteinwirkungen eingesetzt werden, im Reinigungsmodus nicht diesem, sondern einem anderen, wie oben beschriebenen Zweck dienen.

Gemäß einem weiteren Aspekt betrifft die Erfindung eine Recheneinheit zum Reinigen einer Gehäuseoberfläche einer medizinischen Bildgebungsanlage aufweisend Mittel zum Durchführen eines erfindungsgemäßen Verfahrens. Vorteilhaft ist die Recheneinheit in die medizinische Bildgebungsanlage integriert. Alternativ kann die Recheneinheit auch entfernt bzw. abgelegen angeordnet sein. Insbesondere ist die Recheneinheit mit erfindungsgemäßen Sensorelementen, Leuchtelementen und/oder einer Ausgabeeinheit zum Datenaustausch verbunden und eingerichtet, von den Sensorelementen empfangene Signale zu verarbeiten und in Abhängigkeit davon Steuersignale für die Leuchtelemente und/oder die Ausgabeeinheit zu erzeugen.

Gemäß einem weiteren Aspekt betrifft die Erfindung ein Computerprogramm mit Programmcode, um das erfindungsgemäße Verfahren durchzuführen, wenn das Computerprogramm auf einem Computer ausgeführt wird.

In einem weiteren Aspekt betrifft die Erfindung einen computerlesbaren Datenträger mit Programmcode eines Computerprogramms, um das erfindungsgemäße Verfahren durchzuführen, wenn das Computerprogramm auf einem Computer ausgeführt wird.

In noch einem weiteren Aspekt betrifft die Erfindung eine medizinische Bildgebungsanlage zum Reinigen einer Gehäuseoberfläche einer medizinischen Bildgebungsanlage mit einer erfindungsgemäßen Recheneinheit. Insbesondere handelt es sich bei der Bildgebungsanlage um eine C-Bogen-Röntgenanlage umfassend einen Roboter-Stator.

Gemäß einem bevorzugten Ausführungsbeispiel ist die medizinische Bildgebungsanlage in einem Reinigungsmodus betreibbar.

Gemäß einem weiteren Ausführungsbeispiel umfasst die medizinische Bildgebungsanlage eine Gehäuseoberfläche umfassend wenigstens ein Sensorelement, das ausgebildet ist, einen Reinigungsvorgang in wenigstens einem lokalen Teilbereich der Gehäuseoberfläche zu detektieren.

In einem weiteren Ausführungsbeispiel der Erfindung umfasst die medizinische Bildgebungsanlage eine Gehäuseoberfläche umfassend wenigstens ein Leuchtelement, das ausgebildet ist, für wenigstens einen lokalen Teilbereich anzuzeigen, dass dieser gereinigt wurde.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden. Durch diese Beschreibung erfolgt keine Beschränkung der Erfindung auf diese Ausführungsbeispiele. In verschiedenen Figuren sind gleiche Komponenten mit identischen Bezugszeichen versehen. Die Figuren sind in der Regel nicht maßstäblich. Es zeigen:
- FIG 1: eine Ansicht einer medizinischen Bildgebungsanlage in Form eines C-Bogen-Röntgengeräts gemäß einem Ausführungsbeispiel der vorliegenden Erfindung,
- FIG 2: ein schematisches Ablaufdiagramm eines erfindungsgemäßen Verfahrens in einem Ausführungsbeispiel,
- FIG 3: einen Anzeigemonitor einer Bildgebungsanlage mit einer Veranschaulichung eines Reinigungsplans gemäß einem Ausführungsbeispiel der Erfindung,
- FIG 4: einen Anzeigemonitor einer Bildgebungsanlage sowie Teile der Anlage mit einer Veranschaulichung einer erfindungsgemäßen Anzeige eines Reinigungsfortschritts gemäß einem Ausführungsbeispiel der Erfindung, und
- FIG 5: einen Anzeigemonitor einer Bildgebungsanlage sowie Teile der Anlage mit einer Veranschaulichung der erfindungsgemäßen Anzeige eines Reinigungsfort-schritts gemäß einem weiteren Ausführungsbeispiel der Erfindung.

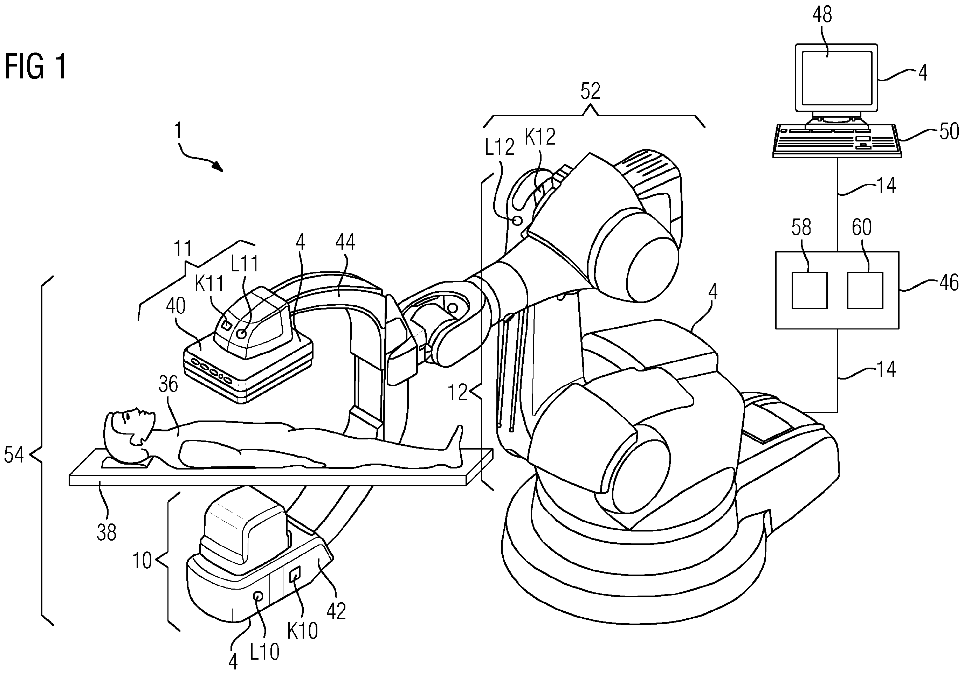

**Figur 1** zeigt eine erfindungsgemäße medizinische Bildgebungsanlage 1 in einem Ausführungsbeispiel. Hier handelt es sich um ein monoplanes angiographisches Röntgensystem 1 mit einem Ständer 52 in Form eines sechsachsigen Knickarmroboters. Das Röntgensystem 1 umfasst eine Vielzahl von Komponenten 52, 44, 42, 40, 38. Bspw. wird eine Komponente in Form eines C-Bogens 44 von der Komponente 52, dem Ständer gehalten. An den Enden des C-Bogens sind weitere Komponenten in Form einer Röntgenstrahlungsquelle, beispielsweise ein Röntgenstrahler 42 mit Röntgenröhre und Kollimator, und ein Röntgenbilddetektor bzw. Röntgendetektor 40 als Aufnahmeeinheit 54 angebracht. Das erfindungsgemäße angiographische Röntgensystem 1 ist insbesondere um Drehzentren und Drehachsen in der C-Bogen-Ebene des Röntgenbilddetektors 40 drehbar. Im Strahlengang des Röntgenstrahlers 42 befindet sich auf einer Patientenliege 38 ein zu untersuchender Patient 36 als Untersuchungsobjekt.

Es wird ohne Beschränkung der Allgemeinheit von einem Patienten als Untersuchungsobjekt ausgegangen, wobei es sich meist um einen Menschen handelt. Grundsätzlich kann der Patient auch ein Tier sein. Die beiden Begriffe "Untersuchungsobjekt" und "Patient" sind synonym verwendet. Das Untersuchungsobjekt kann alternativ eine Pflanze oder ein nicht-lebender Gegenstand, z.B. ein historisches Artefakt oder dergleichen sein.

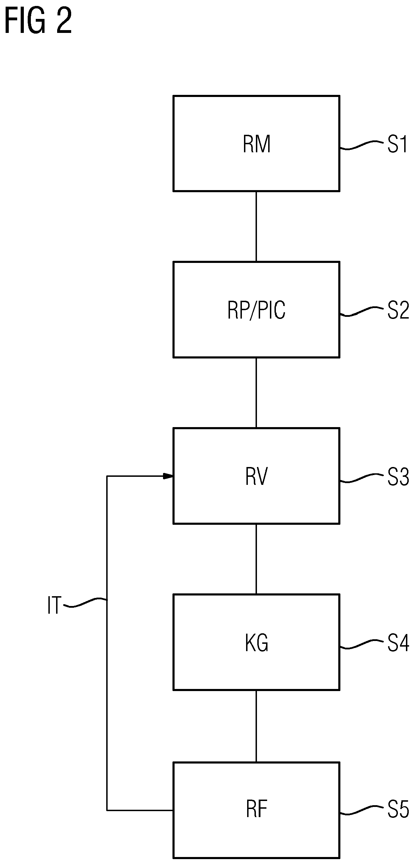

Die Blenden, Gehäuseteile bzw. Verkleidungen der einzelnen Komponenten wie bspw. Röntgenstrahler 42 und Röntgendetektor 40 bilden zusammen nach außen die äußere Gehäuseoberfläche 4, die mittels erfindungsgemäßem Verfahren gereinigt wird, wie weiter unten mit Bezug zu Figur 2 näher erläutert. Zu diesem Zweck ist die äußere Gehäuseoberfläche 4 in verschiedene, also mehrere, wenigstens zwei lokale Teilbereiche 10, 11, 12 unterteilt, hier bspw. gebildet durch das Cover 11 des Röntgenbilddetektors 40, das Cover 10 der Röntgenstrahlenquelle 42 sowie einem Abschnitt 12 des Roboter-Ständers 52. Die hier dargestellten Teilbereiche sind lediglich exemplarisch und nicht abschließend. Insbesondere können die lokalen Teilbereiche auch zusammenhängende, also direkt benachbarte Teilflächen der Gehäuseoberfläche 4 sein. Grundsätzlich ist die Anzahl, Form und/oder Größe der lokalen Teilbereiche 10, 11, 12 beliebig und abhängig von der Form bzw. der Art der Bildgebungsanlage 1 selbst. Insbesondere kann die Gesamtheit aller lokalen Teilbereiche die gesamte Gehäuseoberfläche 4 der Bildgebungsanlage 1 ausmachen. Die erfindungsgemäße Bildgebungsanlage 1 umfasst eine Vielzahl von Sensorelementen, bspw. K10, K11, K12, die in, an, auf, unter der Gehäuseoberfläche 4 verbaut bzw. montiert sind. Die Sensorelemente K10, K11, K12 stehen exemplarisch für eine beliebige Anzahl von Sensorelementen der Bildgebungsanlage 1. Die Sensorelemente K10, K11, K12 sind jeweils in einem lokalen Teilbereich 10, 11, 12 angeordnet. Alternativ kann ein lokaler Teilbereich auch mehrere, also mindestens zwei Sensorelemente umfassen. Die Sensorelemente K10, K11, K12 sind als Sensoren ausgebildet, die eine auf die äußere Gehäuseoberfläche ausgeübte Kraft bzw. eine dieselbe repräsentierende Kenngröße detektieren bzw. erkennen können, welche während eines Reinigens eines lokalen Teilbereichs 10, 11, 12 auf die Gehäuseoberfläche 4 ausgeübt wird. So können die Sensoren K10 und K11 bevorzugt als Dehnungsmessstreifen ausgebildet sein, welche in das Covermaterial eingegossen bzw. eingelassen sind. Der Sensor K12 ist bspw. in bzw. nahe zu einem Knick-Gelenk des Roboter-Ständers 52 in der Gehäuseoberfläche 4 angeordnet und als Drehmomentsensor ausgebildet. Insbesondere können die Sensorelemente K10, K11, K12 als Sensoren der Bildgebungsanlage 1 ausgebildet sein, die in einem anderen Betriebsmodus der Bildgebungsanlage 1, bspw. dem Bilddatenerfassungsmodus, eingesetzt werden, um unerwünschte Bewegung bzw. Berührungen während des Ausführens einer vorab festgelegten Bewegung der Bildgebungsanlage 1 oder einzelner ihrer Komponenten zu erfassen und ggf. die Bewegung aus Sicherheitsgründen sofort zu stoppen.

Die erfindungsgemäße Bildgebungsanlage 1 umfasst darüber hinaus eine Vielzahl von Leuchtelementen L10, L11, L12 in Form von LED- oder OLED-Leuchten. die ebenfalls in, an oder auf der Gehäuseoberfläche 4 verbaut bzw. montiert sind. Die Leuchtelemente L10, L11, L12 stehen ebenfalls exemplarisch für eine beliebige Anzahl von Leuchtelementen der Bildgebungsanlage 1. Die Leuchtelemente L10, L11, L12 sind ebenfalls jeweils in einem lokalen Teilbereich 10, 11, 12 angeordnet und bevorzugt jeweils in räumlicher Nähe, bspw. in einem Abstand von wenigen Zentimetern zu einem entsprechenden Sensorelement K10, K11, K12 platziert. Die Leuchtelemente L10, L11, L12 dienen einer erfindungsgemäßen, optischen Anzeige, dass eine Krafteinwirkung an einem ihr zugeordneten Sensorelement registriert und damit ein Reinigungsprozess des entsprechenden lokalen Teilbereichs 10, 11, 12 erfolgt ist. Selbstredend kann ein lokaler Teilbereich auch mehrere, also mindestens zwei Sensorelemente umfassen. Es können auch mehrere Sensorelemente zu einem Leuchtelement zugeordnet sein und umgekehrt.

Sämtliche Sensorelemente K10, K11, K12 sowie Leuchtelemente L10, L11, L12 können zum Datenaustausch mit einer Steuereinheit 46 der Bildgebungsanlage 1 in bekannter Weise kabellos oder kabelgebunden über Verbindung 14 verbunden sein. Über diese Verbindung 14 können die Leuchtelemente L10, L11, L12 mit durch die Steuereinheit 46 generierten Steuersignalen angesteuert werden und/oder die Sensorelemente K10, K11, K12 können Signale bzgl. der Detektion einer Kenngröße an die Steuereinheit 46 übertragen.

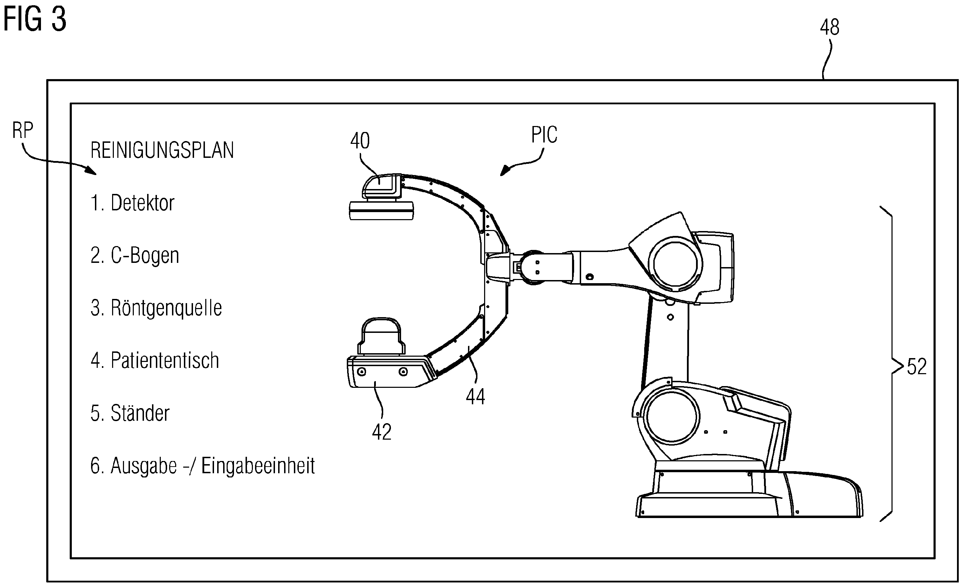

Die Steuereinheit 46 kann als eigenständige Steuereinheit oder als Teil einer Steuereinheit des angiographischen Röntgensystems 1 ausgebildet sein. Die Steuereinheit 46 steht mit einer Ausgabeeinheit 48 und einer Eingabeeinheit 50 über eine weitere Verbindung 14 in Datenkommunikation. Die Ausgabeeinheit 48 dient beispielsweise zur graphischen Anzeige von Verfahrenszuständen des erfindungsgemäßen Verfahrens, wie in den **Figuren 3** bis **5** näher gezeigt. Sie kann aber auch Instruktionen für einen Nutzer, insbesondere Reinigungspersonal, anzeigen, bspw. einen vollständigen Reinigungsplan oder eine detaillierte Reinigungsanleitung für eine bestimmte Komponente der Bildgebungsanlage 1. Die Eingabeeinheit 50 dient beispielsweise zum Eingeben bzw. Empfangen einer Eingabe bzgl. des Startens eines Reinigungsmodus durch den Nutzer zu einem bestimmten Zeitpunkt und/oder der bspw. menüartigen Auswahl von gewünschter, anzuzeigender Information. Bei der Ausgabe-einheit 48 kann es sich beispielsweise um einen LCD-, Plasma-oder OLED-Bildschirm handeln. Es kann sich weiterhin um einen berührungsempfindlichen Bildschirm handeln, welcher auch als Eingabeeinheit 50 ausgebildet ist. Bei der Eingabeeinheit 50 handelt es sich beispielsweise um eine Tastatur, eine Maus, einen sogenannten "Touch-Screen" um ein Mikrofon zur Spracheingabe und/oder einen Fußschalter. Die Eingabeeinheit 50 kann auch eingerichtet sein, um Bewegungen eines Benutzers zu erkennen und in entsprechende Befehle zu übersetzen.

Die Steuereinheit 46 steht auch mit der Aufnahmeeinheit 54 zum Datenaustausch in Verbindung 14. Es können zum Beispiel Steuersignale zur Ausgabe von Leuchtsignalen in Abhängigkeit von durch die Sensorelemente K10, K11, K12 detektierten Kenngrößen für die Leuchtelemente L10, L11, L12, Steuersignale zur Ausgabe von akustischen Warnsignalen oder Steuersignale zum Unterbrechen oder Stoppen einer Bildmessung und/oder zum Stoppen einer dabei ausgeführten Bewegung der Bildgebungsanlage 1 übertragen werden. Die Datenverbindungen 14 sind jeweils in bekannter Weise kabelgebunden oder kabellos realisiert.

Die Steuereinheit 46 umfasst eine Recheneinheit 58, die bspw. dazu eingerichtet ist Steuerbefehle bzw. -signale für die Bildgebungsanlage 1, insbesondere für die Leuchtelemente L10, L11, L12 und/oder für die Ausgabeeinheit 48 zu erzeugen, sodass mittels derselben eine erfindungsgemäße Anzeige bzgl. eines Reinigungsfortschritts erfolgt. Entsprechend kann die Recheneinheit 58 mit einem ebenfalls von der Steuereinheit 46 umfassten Speicher 60 in Datenkommunikation stehen. Darin können bspw. Zuordnungsvorschriften hinterlegt sein, welches wenigstens eine Leuchtelement angesteuert werden muss, wenn ein bestimmtes Sensorelement eine Kenngröße detektiert hat. Alternativ oder zusätzlich können mittels Ausgabeeinheit 48 anzuzeigende Informationen, bspw. der Reinigungsplan, in dem Speicher 60 abrufbar hinterlegt sein und mittels Recheneinheit 58 zur Anzeige gebracht werden, bspw. wenn die Recheneinheit 58 den Start eines Reinigungsmodus der Bildgebungsanlage 1 registriert hat.

Alle genannten Einheiten können auch körperlich oder funktional mit einander verbunden sein.

Die Recheneinheit 58 kann mit einem computerlesbaren Datenträger zusammenwirken, insbesondere, um durch ein Computerprogramm mit Programmcode ein erfindungsgemäßes Verfahren durchzuführen. Weiterhin kann das Computerprogramm auf dem maschinenlesbaren Träger abrufbar gespeichert sein. Insbesondere kann es sich bei dem maschinenlesbaren Träger um eine CD, DVD, Blu-Ray Disc, einen Memory-Stick oder eine Festplatte handeln. Die Recheneinheit 58 kann in Form von Hard- oder in Form von Software ausgebildet sein. Beispielsweise ist die Recheneinheit 58 als ein sogenanntes FPGA (Akronym für das englischsprachige "Field Programmable Gate Array") ausgebildet oder umfasst eine arithmetische Logikeinheit.

In dem hier gezeigten Beispiel ist in dem Speicher 60 der Steuereinheit 46 wenigstens ein Computerprogramm gespeichert, welches alle Verfahrensschritte des erfindungsgemäßen Verfahrens durchführt, wenn das Computerprogramm auf dem Computer ausgeführt wird. Das Computerprogramm zur Ausführung der Verfahrensschritte des erfindungsgemäßen Verfahrens umfasst Programmcode. Weiterhin kann das Computerprogramm als ausführbare Datei ausgebildet sein und/oder auf einem anderen Rechensystem. Beispielsweise kann die medizinische Bildgebungsmodalität 1 so ausgelegt sein, dass die Recheneinheit 58 das Computerprogramm zum Ausführen des erfindungsgemäßen Verfahrens über ein Intranet oder über das Internet in seinen internen Arbeitsspeicher lädt.

**Figur 2** zeigt ein schematisches Ablaufdiagramm eines erfindungsgemäßen Verfahrens in einem Ausführungsbeispiel. Das Verfahren gemäß Ausführungsbeispiel umfasst eine Vielzahl von Schritten S1 bis S5. Diese sind zum Teil optional. Das Verfahren gemäß diesem Ausführungsbeispiel kann zumindest teilweise in einer Iterationsschleifen IT durchlaufen werden. Das Verfahren kann bspw. von der Bildgebungsanlage gemäß Figur 1 ausgeführt werden.

In einem ersten Verfahrensschritt S1 erfolgt eine Aktivierung eines Reinigungsmodus RM der Bildgebungsanlage 1. Dieses Aktivierung bzw. dieses Starten des Reinigungsmodus RM erfolgt hier durch einen Nutzer, insbesondere Reinigungspersonal. Dazu kann dieser bspw. eine entsprechende Eingabe per Eingabeeinheit 50 vornehmen. Die Aktivierung kann alternativ auch mittels Zeitschaltuhr automatisch zu einer vorab festgelegten Zeit erfolgen. Der Reinigungsmodus RM entspricht einem Betriebsmodus, in dem die Bildgebungsanlage 1 für eine Reinigung konfiguriert ist. In diesem Betriebsmodus werden bspw. durch Sensorelemente K10, K11, K12 in Antwort auf durch diese erfasste Kenngrößen erzeugte Sensorsignale ausschließlich für die Feststellung eines Reinigungsfortschrittes in Bezug auf verschiedene lokale Teilbereiche 10, 11, 12 der äußeren Gehäuseoberfläche 4 der Bildgebungsanlage 1 ausgewertet. Darüber hinaus ist die Bildgebungsanlage 1 nur im Reinigungsmodus RM eingerichtet, Informationen bzgl. eines Reinigungsprozesses, bspw. einen Reinigungsplan RP aus dem Speicher 60 abzurufen und/oder auf ihrer Ausgabeeinheit 48 anzuzeigen.

In einem zweiten Verfahrensschritt S2, der optional ist, wird durch das Starten des Reinigungsmodus RM auch eine Anzeige des Reinigungsplans RP über die Ausgabeeinheit 48 beginnen. Der Reinigungsplan RP kann bspw. wie in **Figur 3** dargestellt, eine listenartige Aufstellung verschiedener Reinigungsschritte 1., 2., 3., usw. in Bezug auf verschiedene Komponenten der Anlage 1 bzw. verschiedene lokale Teilbereiche der Gehäuseoberfläche 4 umfassen. Diese können eine beliebige oder zumindest teilweise festgelegte Reihenfolge aufweisen. Zusätzlich oder optional kann die Anzeige des Reinigungsplans RP ein Piktogramm PIC der Anlage 1 umfassen, wie ebenfalls in Figur 3 gezeigt, welches die verschiedenen Komponenten der Bildgebungsanlage 1 veranschaulicht. Insgesamt schafft die Anzeige des Reinigungsplans RP zu Beginn des Reinigungsprozesses eine Übersicht aller notwendigen Reinigungsschritte für das Reinigungspersonal, sodass bei entsprechender Sorgfalt des Personals keine Schritte vergessen werden können. Der Reinigungsplan kann zusätzlich detaillierte Instruktionen in Bezug auf jeden einzelnen Reinigungsschritt umfassen, die Hilfestellung für weniger geschultes Personal geben sollen. Diese können initial gleich mit angezeigt werden oder erst auf entsprechende Eingabe durch den Nutzer N. Die Anzeige des Reinigungsplans RP kann dazu bevorzugt interaktiv sein. So kann zudem vorgesehen sein, dass das Reinigungspersonal N bspw. zunächst das Lesen aller Schritte bestätigen muss, bevor der Reinigungsprozess beginnen kann.

In einem dritten Verfahrensschritt S3 erfolgt ein Reinigungsvorgang RV eines lokalen Teilbereichs der Gehäuseoberfläche 4 durch den Nutzer N entsprechend einem per Reinigungsplan RP vorgesehen Reinigungsschritt. Bspw. beginnt der Nutzer N entsprechend Reinigungsschritt 1. mit der Reinigung des lokalen Teilbereichs 11, der durch das Cover des Detektors 40 gebildet wird. Die Reinigung erfolgt bspw. mittels Wischbewegungen des Nutzers N mit einem Reinigungstuch direkt auf bzw. entlang der Gehäuseoberfläche 4 in dem lokalen Teilbereich. Andere Reinigungsmaßnahmen sind ebenfalls möglich und im Sinne der Erfindung. Der Reinigungsvorgang kann zumindest bei manchen Reinigungsschritten auch ein Verstellen, Verdrehen oder Verkippen oder dergleichen einzelner Komponenten umfassen, bspw. ein Verstellen des C-Bogens 44 um wenigstens eines der Gelenke des Roboter-Ständers 52. Beides verursacht, entweder durch den Benutzer oder die Bewegung der Anlage 1 oder ihrer Komponenten, eine Krafteinwirkung auf die Gehäuseoberfläche 4. Diese ist messbar, sofern der Benutzer bzw. die Bewegung der Anlage 1 beim Reinigen wenigstens ein Sensorelement K10, K11, K12 berührt bzw. betrifft.

Insofern wird in einem vierten Verfahrensschritt S4 ein Reinigungsvorgang detektiert, indem wenigstens eine eine Krafteinwirkung repräsentierende Kenngröße KG mittels wenigstens eines Sensorelements K10, K11, K12 in dem lokalen Teilbereich erfasst wird. Konkret misst also Sensorelement K11, der in dem lokalen Teilbereich des Detektors 40 angeordnet ist, eine Kenngröße. Dieser Verfahrensschritt umfasst auch, durch das Sensorelement ein entsprechendes Sensorsignal zu erzeugen und zusammen mit Sensoridentifikationsdaten an die Recheneinheit 58 zu übertragen.

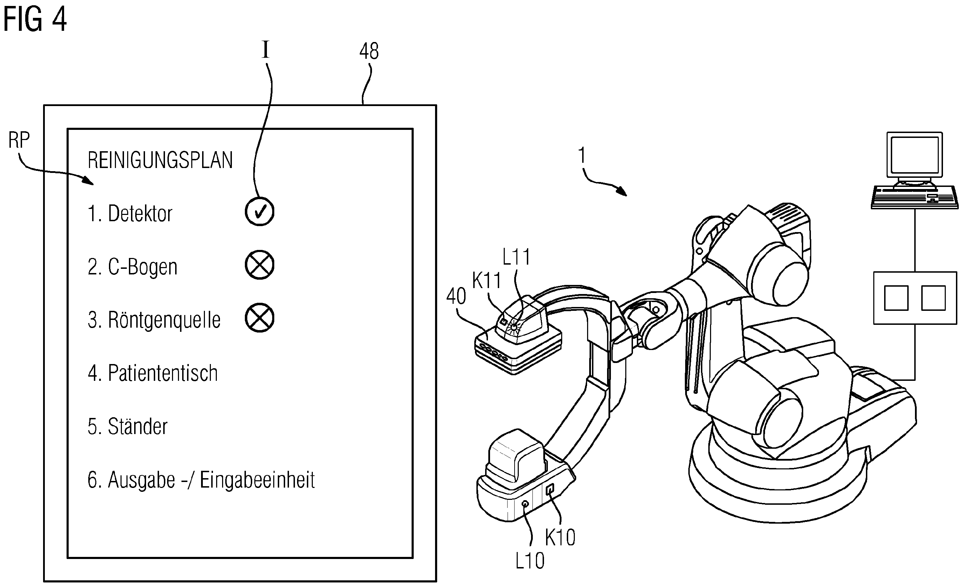

Diese ermittelt nun in einem fünften Verfahrensschritt S5 aus der übertragenen Information bspw. mit entsprechend im Speicher 60 hinterlegten Look-Up-Tabellen, welcher lokale Teilbereich gereinigt wurde und ggf. welches Leuchtelement L10, L11, L12 diesem lokalen Teilbereich zugeordnet ist. Entsprechend erzeugt die Recheneinheit 48 ein Steuersignal für wenigstens ein identifiziertes Leuchtelement und überträgt dieses an dasselbe. Zusätzlich oder alternativ kann vorgesehen sein, dass die Recheneinheit 48 weitere Steuersignale für die Ausgabeeinheit erzeugt und diese dorthin überträgt. Entsprechend erfolgt in diesem Verfahrensschritt eine erfindungsgemäße Anzeige einer Information bezüglich des gereinigten, lokalen Teilbereichs 11. Mit anderen Worten erfolgt in diesem Verfahrensschritt eine Anzeige eines Reinigungsfortschritts RF. Diese Anzeige erfolgt einerseits auf der Gehäuseoberfläche 4 selbst, und zwar bevorzugt in dem lokalen Teilbereich 11 durch das wenigstens eine, per Recheneinheit 48 angesteuerte Leuchtelement L11 in Form bspw. einer LED. Diese leuchtet, wie in Figur 4 dargestellt und stellt somit schnell und intuitiv für den Nutzer einsehbar dar, dass der Detektor 40 bereits gereinigt ist. Zusätzlich oder alternativ kann die Ausgabeeinheit 48 in Antwort auf die erhaltenen Steuersignale mittels eines Indikators I, bevorzugt Piktogramm-artig den Reinigungsfortschritt RF anzeigen, wie ebenfalls in Figur 4 verdeutlicht. Hier ist der Indikator I als Häkchen hinter dem Reinigungsschritt 1. ausgebildet. Andere Ausgestaltungsvarianten sind ebenfalls möglich.

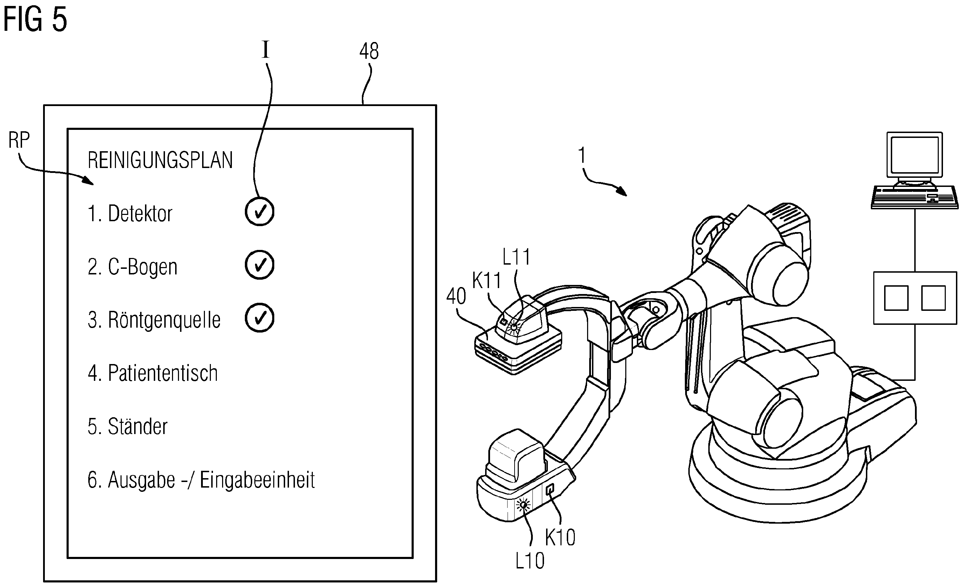

Die Schritte S3 bis S5 können nun im Rahmen des Reinigungsprozesses für jeden weiteren Reinigungsschritt bzw. für jeden weiteren lokalen Teilbereich über die Iterationsschleife IT wiederholt werden, solange, bis alle erforderlichen Komponenten der Bildgebungsanlage 1 oder die gesamte äußere Gehäusefläche gereinigt wurden. So umfasst nach mehrmaligem Durchlaufen der Iterationsschleife IT die Anzeige des Reinigungsfortschritts RF gemäß Figur 5 mehrere aktive Leuchtelemente L10 bis L12 (L12 nicht dargestellt) an der Anlage 1 und ggf. optional werden mehrere Indikatoren I in Form von Häkchen auf der Ausgabeeinheit 48 angezeigt.

Zusammenfassend wird mit der vorliegenden Erfindung ein verbesserter Reinigungsprozess für Bildgebungsanlagen bereitgestellt. Über die Nutzung bereits in die äußere Gehäuseoberfläche 4 integrierter Sensoren zur Erfassung von Krafteinwirkungen bzw. Bewegungen kann in einem Reinigungsmodus eine schnelle und intuitive Anzeige für Reinigungspersonal erfolgen, welche Geräteteile bzw. Komponenten schon sauber sind. Im Reinigungsmodus ist aus Sicherheitsgründen vorteilhaft eine übrige Bedienbarkeit der Bildgebungsanlage 1 ausgeschlossen und die mittels Sensoren erfassten Größen werden ausschließlich für die erfindungsgemäße Anzeige des Reinigungsfortschrittes RF heran gezogen. Insofern stellt die vorliegende Erfindung ein, intelligentes Cover' bereit, welches des Reinigungsfortschritt RF selber überwacht und anzeigt. Über die zusätzliche, optionale Veranschaulichung des Reinigungsfortschritts RF über die Ausgabeeinheit 48, hat das Reinigungspersonal eine Checklisten-artige Prüfübersicht des gesamten Reinigungsprozesses für die Anlage 1.

Wo noch nicht explizit geschehen, jedoch sinnvoll und im Sinne der Erfindung, können einzelne Ausführungsbeispiele, einzelne ihrer Teilaspekte oder Merkmale mit einander kombiniert bzw. ausgetauscht werden, ohne den Rahmen der hiesigen Erfindung zu verlassen. Mit Bezug zu einem Ausführungsbeispiel beschriebene Vorteile der Erfindung treffen ohne explizite Nennung, wo übertragbar, auch auf andere Ausführungsbeispiele zu.

## Patentansprüche

1. Verfahren zum Reinigen einer Gehäuseoberfläche (4) einer medizinischen Bildgebungsanlage (1) umfassend folgende Schritte:
- Aktivieren (S1) eines Reinigungsmodus (RM) an der Bildgebungsanlage,
- Detektieren (S4) eines Reinigungsvorgangs (RV) in wenigstens einem lokalen Teilbereich (10, 11, 12) der Gehäuseoberfläche, und
- Anzeigen (S5) einer Information bezüglich des wenigstens einen, lokalen Teilbereichs, der gereinigt wurde.

2. Verfahren nach Anspruch 1, wobei das Aktivieren des Reinigungsmodus durch einen Benutzer (N) erfolgt.

3. Verfahren nach einem der vorhergehenden Ansprüche, wobei das Aktivieren des Reinigungsmodus ein Anzeigen (S2) eines Reinigungsplans (RP) für die Bildgebungsanlage auf einer Ausgabeeinheit (48) der Bildgebungsanlage umfasst.

4. Verfahren nach Anspruch 3, wobei das Detektieren des Reinigungsvorgangs umfasst, mittels wenigstens eines in räumlicher Nähe zu der Gehäuseoberfläche des lokalen Teilbereichs angeordneten Sensorelements (K10, K11, K12) eine durch den Reinigungsvorgang auf die Gehäuseoberfläche ausgeübte Kraft zu detektieren.

5. Verfahren nach Anspruch 4, wobei das Detektieren umfasst, eine der ausgeübten Kraft entsprechende Kenngröße (KG) aus der Gruppe der folgenden Kenngrößen (KG) zu erfassen: Druck, Spannung, Strom, Kapazität, Widerstand, Kraft, Drehmoment.

6. Verfahren nach einem der vorhergehenden Ansprüche, wobei das Anzeigen der Information bezüglich des gereinigten lokalen Teilbereichs optisch erfolgt.

7. Verfahren nach Anspruch 6, wobei das Anzeigen der Information bezüglich des gereinigten lokalen Teilbereichs über die Gehäuseoberfläche und/oder über eine Ausgabeeinheit der Bildgebungsanlage erfolgt.

8. Verfahren nach einem der vorhergehenden Ansprüche, wobei ein Betrieb der Bildgebungsanlage im Reinigungsmodus eine Bedienbarkeit der Bildgebungsanlage im Sinne einer Bilddatenerfassung sperrt.

9. Recheneinheit (58) zum Reinigen einer Gehäuseoberfläche (4) einer medizinischen Bildgebungsanlage (1) aufweisend Mittel zum Durchführen eines Verfahrens gemäß einem der Ansprüche 1 bis 8.

10. Computerprogramm mit Programmcode, um das Verfahren nach einem der Ansprüche 1 bis 8 durchzuführen, wenn das Computerprogramm auf einem Computer ausgeführt wird.

11. Computerlesbarer Datenträger mit Programmcode eines Computerprogramms, um das Verfahren nach einem der Ansprüche 1 bis 8 durchzuführen, wenn das Computerprogramm auf einem Computer ausgeführt wird.

12. Medizinische Bildgebungsanlage (1) zum Reinigen einer Gehäuseoberfläche (4) der medizinischen Bildgebungsanlage mit einer Recheneinheit (58) nach Anspruch 10.

13. Medizinische Bildgebungsanlage nach Anspruch 12, die in einem Reinigungsmodus (RM) betreibbar ist.

14. Medizinische Bildgebungsanlage nach einem der Ansprüche 12 oder 13, mit einer Gehäuseoberfläche umfassend wenigstens ein Sensorelement (K10, K11, K12), das ausgebildet ist, einen Reinigungsvorgang (RV) in wenigstens einem lokalen Teilbereich (10, 11, 12) der Gehäuseoberfläche zu detektieren.

15. Medizinische Bildgebungsanlage nach einem der Ansprüche 12 bis 14, mit einer Gehäuseoberfläche umfassend wenigstens ein Leuchtelement (L10, L11, L12), das ausgebildet ist, für wenigstens einen lokalen Teilbereich anzuzeigen, dass dieser gereinigt wurde.
